# EUROPÄISCHE PATENTANMELDUNG

(11) **EP 1 710 565 A1**
(43) Veröffentlichungstag der Anmeldung: **11.10.2006**
(21) Anmeldenummer: 05007338.6
(22) Anmeldetag: 05.04.2005
(51) Int. Cl.: G01N 21/86, G01N 35/00

(54) **Mobiles optisches Diagnosesystem**

(71) Anmelder: F. HOFFMANN-LA ROCHE AG, 4070 Basel (CH); Roche Diagnostics GmbH, 68305 Mannheim (DE)
(72) Erfinder: Schmid, Wilfried, 68185 Mannheim (DE)
(74) Vertreter: Isenbruck, Günter

(57) **Zusammenfassung**

In vielen Bereichen der medizinischen Diagnostik, beispielsweise zur Blutzuckermessung, werden optische Testelemente (112) eingesetzt. Es wird ein System (110; 410) zur Auswertung derartiger Testelemente (112) vorgeschlagen, welches ein Testelement (112) und ein mobiles Datenübertragungsgerät (120), insbesondere ein Mobiltelefon (120), mit einer Kamera (122), sowie optional eine Haltevorrichtung (126) aufweist. Die Haltevorrichtung (126) weist mindestens eine Halterung (138) zur Aufnahme eines Testelements (112) auf, sowie eine Verbindungsvorrichtung (128) zur räumlichen Fixierung und/oder Positionierung der Haltevorrichtung (126) zur Kamera (122) des mobilen Datenübertragungsgerätes (120). Nach Applikation einer Probe, beispielsweise eines Bluttropfens, auf das Testelement (112), wird mittels der Kamera (122) eine Bildinformation eines optischen Testfelds (114) des Testelements (112) aufgenommen und mittels eines Auswertungsverfahrens ausgewertet. Optional kann für diese Auswertung ein Auswertungsserver (412) herangezogen werden, welcher mit dem mobilen Datenübertragungsgerätes (120) über ein Mobilfunknetz (414) in Verbindung steht.

## Beschreibung

### Gebiet der Erfindung

Die Erfindung betrifft eine Haltevorrichtung, ein System und ein Verfahren zur Auswertung eines Testelements, beispielsweise eines Testelements zur medizinischen oder chemischen Analyse. Insbesondere ist das System zur chemisch-optisrhen Analyse flüssiger Proben geeignet, insbesondere von Blut, Blutbestandteilen, Speichel oder Urin. Das System kann beispielsweise zur Bestimmung einer Glukosekonzentration, Cholesterinlconzentration, Alkoholkonzentration oder anderer chemischer Substanzen eingesetzt werden.

### Stand der Technik

In vielen Bereichen der Chemie, Umweltanalytik, Verfahrenstechnik, Biologie, Biochemie und medizinischen Diagnostik spiel die Quantifizierung vorgegebener chemischer Substanzen eine wichtige Rolle. Ein Beispiel von erheblicher praktischer Bedeutung ist die Überwachung von Blutglukosekonzentrationen, wobei jedoch im Bereich der medizinischen Diagnostik zahlreiche weitere Beispiele zu nennen sind, beispielsweise die Bestimmung eines Cholesterinanteils im Blut.

Die Bestimmung von Blutglukosekonzentrationen ist für Diabetiker ein essenzieller Bestandteil des Tagesablaufs. Dabei muss die Blutglukosekonzentration schnell und einfach mehrmals am Tag (typischerweise 2- bis 7-mal) bestimmt werden, um gegebenenfalls entsprechende medizinische Maßnahmen ergreifen zu können. Um den Tagesablauf des Diabetikers nicht mehr als nötig einzuschränken, werden dabei häufig entsprechende mobile Geräte eingesetzt, welche einfach zu transportieren und zu handhaben sein sollten, so dass die Messung der Blutglukosekonzentration beispielsweise am Arbeitsplatz oder auch in der Freizeit erfolgen kann. Derzeit sind verschiedene mobile Geräte am Markt, welche teilweise nach unterschiedlichen Meßmethoden und unter Verwendung unterschiedlicher Diagnoseverfahren funktionieren. Ein erstes Messverfahren beruht beispielsweise auf einer elektrochemischen Meßmethode, bei welchem eine Blutprobe auf eine mit Enzymen und Mediatoren beschichtete Elektrode appliziert wird. Entsprechende Teststreifen für dieses elektrochemische Messverfahren sind beispielsweise in US 5,286,362 beschrieben.

Eine zweite bekannte Meßmethode verwendet optische Messverfahren. Diese Messverfahren beruhen im Wesentlichen darauf, dass die zu detektierende Substanz, im vorliegenden Fall beispielsweise Glukose, mit bestimmten Nachweisreagenzien reagieren kann, wobei eine Farbänderung des Reaktionsgemisches eintritt. Üblicherweise werden für diesen optischen Nachweis Testelemente, beispielsweise in Form von Teststreifen oder Testplatten verwendet, auf welchen die Nachweisreagenzien und ggf. Trägennedien aufgebracht sind. Derartige Systeme sind aus dem Stand der Technik weithin bekannt, beispielsweise aus CA 2,050,677, in welcher eine Separationsmatrix mit verschiedenen Testreagenzien beschrieben wird, welche bei Reaktion mit Glukose, Cholesterin, Alkohol oder ähnlichen chemischen Substanzen entsprechende farbige Produkte bilden, welche optisch nachgewiesen werden können. In der DE 698 15 207 T2 wird ein Verfahren zum Messen von Cholesterin in einer Lebenendkörperprobe beschrieben, bei welchem ein Reaktionsprodukt der Probe mit einem Reagenz, welches ein bestimmtes Enzym umfasst, optisch gemessen wird.

In DE 695 24 108 T2 wird ein Verfahren zur optischen Messung der Konzentration von Glukose in Gesamtblut beschrieben, bei welchem Teststreifen mit einer Reaktionszone verwendet werden. Das Reflektionsvermögen der Reaktionszone variiert als eine Funktion einer Menge an Analyt (beispielsweise Glukose), welches in einer aufgetragenen Flüssigkeit vorhanden ist. Insbesondere wird dies dadurch erreicht, dass das Analyt, falls vorhanden, mit Reaktanden reagiert, um eine Farbveränderung der Reaktionszone zu erzeugen. Weiterhin umfasst der Teststreifen eine optisch sichtbare Standardzone, welche als kalibrierter Standard fungiert, so dass die Konzentration an fraglichem Analyt als eine Funktion des Standardreflektionsvermögens und des Reaktionszonenreflektionsvermögens bestimmt werden kann.

Weiterhin sind aus dem Stand der Technik verschiedene Verfahren bekannt, mittels derer optische Testelemente ausgewertet werden können. Neben einer Auswertung, bei der ein Benutzer das Testelement betrachtet und entsprechend eine Farbveränderung beurteilt (beispielsweise anhand einer Farbskala), sind auch automatische Auswertungsverfahren bekannt, bei denen ein Testelement, beispielsweise ein Teststreifen, automatisch mittels einer entsprechenden Vorrichtung ausgelesen wird. So ist beispielsweise aus der DE 695 24 108 T2 ein Teststreifenlesegerät bekannt, bei welchem der oben beschriebene Teststreifen mit Kalibrationsstandard mittels eines optischen Reflektionsverfahrens ausgelesen wird. Das beschriebene Lesegerät zeigt dann unmittelbar eine Analytgegenwart oder einer Konzentration des Analyts, insbesondere von Blutglukose, an.

Weiterhin sind aus dem Stand der Technik auch Vorrichtungen bekannt, bei welchen Kameras zur Auswertung von optischen Testsubstraten eingesetzt werden, So beschreibt die WO 94/19767 ein System, welches automatisch eine Auftragungsposition eines Analyts auf die Oberfläche eines Testsubstrates, welches ein entsprechendes Reagenz enthält, bestimmt. Dabei wird eine Schwärzung bzw. Verfärbung des Testsubstrats in Folge einer Reaktion des Reagenz mit dem Analyten festgestellt. Weiterhin erfasst die eingesetzte Kamera auch einen Kalibrationsbereich auf dem Testsubstrat abseits von dem Reaktionsbereich, welcher zur Kalibration der von der Kamera erzeugten Bilddaten dient.

Auch aus der EP 646 784 A1 ist ein entsprechendes System bekannt, welches eine Video-Bildgebung oder eine Kamera umfasst und welches zur Auswertung von Teststreifen eingesetzt werden kann, wobei ggf. auch Farbinformation ausgewertet wird. Ein ähnliches System zur Auswertung eines chemischen Diagnostikelements ist auch in der EP 953 149 B1 beschrieben. Hierbei wird anstelle einer Kamera, zur Erfassung eines digitalen Bildes ein digitales Bilderfassungssystem, beispielsweise ein optischer Flachbettscanner, eingesetzt. Anschließend wird mittels eines Computers eine Farbanalyse der Bilddaten eines Testelements durchgeführt und so das Testelement ausgewertet. Das in der EP 953 149 B1 beschriebene System und Verfahren nimmt Bezug auf eine Vielzahl kommerziell erhältlicher Testelemente, insbesondere Teststreifen, zur Konzentrationsbestimmung zahlreicher chemischer Substanzen in einer Vielzahl von Proben, beispielsweise Blut, Urin, Speichel oder Stuhl.

Die aus dem Stand der Technik bekannten Verfahren, bei welchen optische Methoden zur Auswertung von Testelementen verwendet werden, weisen, insbesondere für die tägliche Benutzung durch einen Patienten, zahlreiche Nachteile auf. Einige der im Stand der Technik beschriebenen Vorrichtung sind technisch äußerst aufwändig und daher mit hohen Herstellungskosten verbunden. Insbesondere im Bereich der medizinischen Diagnostik für den täglichen Bedarf einzelner Patienten sind jedoch geringe Anschaffungskosten entsprechender diagnostischer Geräte unerlässlich.

Weiterhin sind viele der aus dem Stand der Technik bekannten Geräte nicht oder nur bedingt als mobile Diagnosegeräte für den täglichen Bedarf geeignet Insbesondere Geräte zur optischen Auswertung von Testelementen erfordern regelmäßig empfindliche Optiken, welche in vielen Fällen den harten Anforderungen, die an ein mobiles Gerät gestellt werden, nicht gewachsen sind. Insbesondere können Erschütterungen oder Eintluss von Luft-feuchtigkeit oder Verschmutzungen die Funktionalität dieser Geräte beeinflussen. Andere aus dem Stand der Technik bekannte Vorrichtungen wiederum, wie beispielsweise Flachbettscanner, sind aufgrund ihres hohen Platzbedarfs für einen Einsatz in mobilen Geräten ungeeignet.

### Aufgabe der Erfindung

Aufgabe der vorliegenden Erfindung ist es daher, ein System und ein Verfahren bereitzustellen, welches die dargestellten Nachteile des Standes der Technik ganz oder teilweise vermeiden. Insbesondere soll ein System und ein Verfahren zur Auswertung eines Testelements bereitgestellt werden, welches einfach zu handhaben, kostengünstig und Platz sparend ist.

### Darstellung der Erfindung

Diese Aufgabe wird durch die Erfindung mit den Merkmalen der unabhängigen Ansprüche gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht

Es wird ein System zur Auswertung eines Testelements, insbesondere eines Testelements zur medizinischen Diagnose, vorgeschlagen sowie eine Haltevorrichtung zur Auswertung eines Testelements und ein Testelement. Weiterhin wird ein Kalibrierelement zum Kalibrieren des erfindungsgemäßen Systems vorgeschlagen sowie ein Verfahren zum Durchführen einer Diagnose, insbesondere zum Durchführen einer Diagnose mittels mindestens eines Testelements.

Die erfindungsgemäße Haltevorrichtung weist mindestens eine Halterung zur Aufnahme mindestens eines Testelements auf. Dieses Testelement, wobei es sich insbesondere um ein Testelement zur medizinischen Analyse handeln kann, vorzugsweise um einen Teststreifen, weist mindestens ein optisches Testfeld auf. Dabei lassen sich grundsätzlich eine Vielzahl von Testelementen mit optischen Testfeldem einsetzen, beispielsweise die eingangs genannten Testelemente, welche aus dem Stand der Technik (z. B. aus CA 2,050,677, DE 6945 24 108 T2 oder EP 953 149 B1) bekannt sind. Die für die erfindungsgemäße Haltevorrichtung in das erfindungsgemäße System eingesetzten Testelemente weisen mindestens ein optisches Testfeld auf Werden mehrere optische Testfelder eingesetzt, so können beispielsweise unterschiedliche Testfelder sensitiv auf unterschiedliche chemische Substanzen reagieren. Insbesondere kann das mindestens eine Testelement derart ausgestaltet sein, dass das mindestens eine Testelement und/oder das mindestens eine optische Testfeld entsprechende Reagenzien aufweist, welche mit mindestens einer vorgegebenen chemischen Substanz, welche detektiert werden soll, reagieren, wobei das mindestens eine Testelement bzw. das entsprechende mindestens eine optische Testfeld, mindestens eine optische Eigenschaft ändert. Insbesondere kann es sich bei dieser optischen Eigenschaft um eine Farbe, ein Reflektionsvermögen und/oder eine Fluoreszenzeigenschaft handeln.

Als besonders vorteilhaft hat es sich erwiesen, wenn das Testelement einen optischen Teststreifen, insbesondere einen Teststreifen zur chemisch-optischen Analyse flüssiger Proben, vorzugsweise Blut und/oder Blutbestandteile und/oder Speichelbestandteilen und/oder Speichel und/oder Urin aufweist. Dabei können beispielsweise die in der EP 953 149 B1 erwähnten Teststreifen und Testsubstrate eingesetzt werden. Auch Endlos-Teststreifen, welche beispielsweise in Rollenform gelagert und eingesetzt werden, sind einsetabar. Insbesondere können dabei Teststreifen eingesetzt werden, welche auf Glukose, insbesondere Blutglukose und/oder Cholesterin und/oder Alkohol und/oder Kreatinin und/oder Ketone sensitiv reagieren, so dass mindestens eine optische Eigenschaft mindestens eines optischen Testfeldes des Teststreifens bei Anwesenheit einer oder mehrere dieser chemischen Substanzen verändert wird. Daneben sind jedoch auch zahlreiche weitere chemische Substanzen denkbar, für deren Bestimmung das erfindungsgemäße System und die erfindungsgemäße Haltevorrichtung sowie das erfindungsgemäße Verfahren eingesetzt werden können.

Die Art und Weise der Applikation einer Probe auf das Testelement kann auf verschiedene Weise ausgestaltet sein und hängt stark von der technischen Ausgestaltung des mindestens einen Testelements ab. Werden beispielsweise Teststreifen verwendet zur Diagnose flüssiger Proben, so lassen sich beispielsweise einzelne Tropfen auf einen Teststreifen applizieren. Diese Applikation kann beispielsweise unmittelbar erfolgen, zum Beispiel indem ein Bluttropfen erzeugt und unmittelbar auf dem Teststreifen abgestreift wird. Auch ein Applizieren mittels einer Pipette ist denkbar. Weiterhin ist es auch denkbar, einen Teststreifen vollständig in eine flüssige Probe einzutauchen, was insbesondere dann vorteilhaft ist, wenn eine ausreichende Menge der flüssigen Probe zur Verfügung steht, Insbesondere ist dies bei Speichel-, Stuhl- oder Urinproben der Fall. Steht hingegen lediglich eine geringe Menge der flüssigen Probe zur Verfiigung, beispielsweise im Falle von Blutglukosemessungen, so ist es vorteilhaft, die benötigte Probenmenge stark zu reduzieren. Kommerziell erhältliche Teststreifen für elektrochemische Messverfahren zur Blutglukosemessung kommen bereits mit Blutmengen unter 1,5 ml aus. So ist es in diesem Fall vorteilhaft, Testelemente, insbesondere Teststreifen einzusetzen, welche mindestens ein Kapillarsystem aufweisen, wobei das mindestens eine Kapillarsystem ausgestaltet ist, um mindestens eine flüssige Probe von mindestens einem Aufbringort zu mindestens einem optischen Testfeld zu leiten. Diese Weiterbildung der Erfindung hat den Vorteil, dass auch für optische Testverfahren geringe Probenmengen ausreichen.

Weiterhin ist es vorteilhaft, wenn das mindestens eine Testelement mindestens eine Referenzeinrichtung zur Erzeugung mindestens einer Referenz, insbesondere einer Farbreferenz und/oder einer Fluoreszenzreferenz und/oder einer Reflektionsreferenz und/oder einer Positionsreferenz und/oder einer Skalierungsreferenz, aufweist. Derartige Referenzen, welche zum Teil beispielsweise aus der DE 695 24 108 T2 bekannt sind, erleichtern die optische Auswertung des Testelements, indem beispielsweise eine Kalibration auf einen Kontrast, Hintergrundlicht, Position des Testelements, Eigenschaften einer Kamera oder ähnliches durchgeführt werden kann. Erfindungsgemäß wird daher weiterhin ein Testelement vorgeschlagen, welches neben mindestens einem optischen Testfeld mindestens eine Referenzeinrichtung zur Erzeugung mindestens einer Referenz aufweist, Bei der mindestens einen Referenz handelt es sich vorzugsweise um eine Farbreferenz und/oder eine Fluoreszenzreferenz und/oder eine Reflexionsreferenz und/oder eine Positionsreferenz und/oder eine Skalierungsreferenz.

Diese Referenzeinrichtung kann beispielsweise ein oder mehrere farbige Felder aufweisen, beispielsweise Felder verschiedener Farben und/oder Felder ein und derselben Farbe mit verschiedenen Farbtiefen (Helligkeit). Auch verschiedene Graustufen können eingesetzt werden. Weiterhin können beispielsweise auch Referenzfelder mit unterschiedlicher Reflektion eingesetzt werden. Außerdem können beispielsweise Positionsmarken eingesetzt werden, welche zum Beispiel die Positionierung des mindestens einen Testelements innerhalb der Haltevorrichtung ermöglichen und/oder beispielsweise auch eine Messung in verschiedenen Positionen auf dem Testelement ermöglichen. So kann eine Positionsmarke die optische Auswertung eines Testelements erheblich erleichtern, da aus der Bildinformation gleichzeitig auch der Ort auf dem Testelement bekannt ist. Weiterhin kann die Referenzeinrichtung beispielsweise auch eine Fluoreszenzreferenz beinhalten, beispielsweise fluoreszierende Felder, so dass beispielsweise auch Fluoreszenzanalysen durchgeführt werden können, wobei eine Kalibration der Analyse, beispielsweise hinsichtlich der Intensität eines-Anregungslichtes anhand der fluoreszierenden Referenzeinrichtung durchgeführt werden kann. Wird eine Skalierungsreferenz eingesetzt, beispielsweise in Form eines auf dem Testelement angeordneten Skalierungsmaßstabs, so kann beispielsweise aus einer Bildinformation, in welcher ein Bild des Skalierungsmaßstabs gespeichert ist, ein Skalierungsfaktor ermittelt werden, mittels dessen die Bildinformation reskaliert werden kann. Auf diese Weise wird insbesondere die Auswertung unabhängig von der Größe eines aufgenommenen Bildbereiches,

Die Halterung zur Aufnahme des mindestens einen Testelements ist dem verwendeten Testelement angepasst. Insbesondere kann diese Halterung einen Schlitz aufweisen, in welchen beispielsweise ein Testelement, beispielsweise ein Teststreifen, eingeschoben und dort gehaltert wird, Auch andere Ausgestaltungen sind denkbar, beispielsweise entsprechende Halterungen mittels eines Federsystems. Derartige Halterungen sind beispielsweise in der DE 695 24 108 T2 beschrieben.

Insbesondere kann die erfindungsgemäße Haltevorrichtung vorzugsweise auch mindestens ein Magazin zur Aufnahme und/oder Ausgabe des mindestens einen Testelements aufweisen, beispielsweise ein Stapel- oder Trommelmagazin zur Aufnahme mehrer Testelemente, vorzugsweise von Teststreifen. Ein derartiges Magazin ist beispielsweise in EP 1 321 769 A1 beschrieben. Vorzugsweise kann dieses mindestens eine Magazin mindestens einen Vorratsbehälter zur Aufnahme und/oder Ausgabe mindestens eines unbenutzten Testelements und/oder mindestens einen Abfallbehälter zur Aufnahme mindestens eines benutzten Testelements aufweisen, Beispielsweise kann das mindestens eine Magazin einen Spulenmechanismus zum Befördern des mindestens einen Teststreifens aufweisen.

Die Halterung kann jedoch weiterhin auch davon abhängen, zu welchem Zeitpunkt und wie die zu analysierende Probe, beispielsweise die flüssige Probe, auf das Testelement appliziert wird. So kann beispielsweise, wenn ein Teststreifen verwendet wird, zunächst die Probe auf den Teststreifen appliziert werden, und dann dieser Teststreifen vom Benutzer in die Halterung eingebracht werden. Vorteilhafterweise kann die Applikation auch erfolgen, während das Testelement schon in die Halterung eingebracht ist, so dass der Patient das Testelement nicht mehr handhaben muss. Beispielsweise kann zu diesem Zweck die Halterung derart ausgestaltet sein, dass das Testelement, insbesondere der Teststreifen, in verschiedenen Positionen gehaltert werden kann, beispielsweise in einer Applikationsposition und einer Messposition. Insbesondere können auch unmittelbar aus dem Magazin Testelemente in die Halterung zugeführt werden, beispielsweise automatisch oder infolge einer Einwirkung durch einen Benutzer. Die Halterung kann auch Bestanteil des Magazins sein oder umgekehrt,

Die Haltevorrichtung ist erfindungsgemäß weiterhin derart ausgestaltet, dass das Testelement in mindestens einer Auswertungsposition positionierbar ist, wobei in der mindestens einen Auswertungsposition das mindestens eine optische Testfeld von einem Sichtbereich mindestens einer Kamera eines mobilen Datenübertragungsgerätes, insbesondere eines Mobiltelefons, erfassbar ist.

Dabei kann die Haltevorrichtung in der Auswertungsposition beispielsweise ein Sichtfenster aufweisen, wobei das Testelement derart positioniert werden kann, dass das mindestens eine optische Testfeld in diesem Sichtfenster positioniert ist und dort vom Sichtbereich der Kamera erfassbar ist. Somit kann dann mittels der Kamera eine Bildinformation aufgenommen werden. Das mobile Datenübertragungsgerät kann beispielsweise bei der Hand gehalten werden. Bevorzugt ist es jedoch, wenn die Haltevorrichtung weiterhin mindestens eine Verbindungseinrichtung aufweist. Diese Verbindungseinrichtung dient zur räumlichen Fixierung und/oder Positionierung der Haltevorrichtung zur Kamera des mobilen Datenübertragungsgerätes. Die räumliche Fixierung kann dabei insbesondere derart ausgestaltet sein, dass in mindestens einer Position des mindestens einen Testelements der Sichtbereich der mindestens einen Kamera mindestens ein optisches Testfeld des mindestens einen Testelements erfasst.

Unter einem Sichtbereich der Kamera ist dabei insbesondere ein maximaler Winkelbereich der Kamera zu verstehen, innerhalb dessen die Kamera oder ggf. eine entsprechende Optik dieser Kamera Lichtstrahlen aufnehmen kann. Dieser Sichtbereich kann fest sein (Fixfokus) oder auch variabel, beispielsweise durch Verwendung eines Zoom-Objektives. Unter "Erfassen" ist insbesondere zu verstehen, dass das mindestens eine optische Testfeld ganz oder teilweise im Sichtbereich der mindestens einen Kamera liegt. Dabei kann der Sichtbereich der mindestens einen Kamera ein einzelnes optisches Testfeld (ganz oder teilweise) oder auch mehrere optische Testfelder (ganz oder teilweise) gleichzeitig erfassen.

Die erfindungsgemäße Haltevorrichtung ermöglicht insbesondere die Auswertung eines Testelements, beispielsweise eines Teststreifens mit optischen Testfeldern, mittels eines einfachen, eine Kamera aufweisenden mobilen Datenübertragungsgerätes. Der Begriff eines mobilen Datenübertragungsgerätes, insbesondere eines Mobiltelefons, ist weit zu fassen. Insbesondere sollen neben Mobiltelefonen auch Kleincomputer, beispielsweise Handhelds oder PDAs mit Datenübertragungsfunktion (insbesondere zur Datenübertragung mittels eines Mobilfunknetzes), erfasst sein. Ein wesentlicher Vorteil der erfindungsgemäßen Lösung besteht also darin, dass für die Auswertung eines Testelements ein elektronisches Gerät, welches von vielen Benutzern bzw. Patienten im täglichen Leben mitgeführt wird, nämlich ein Mobiltelefon mit Kamerafunktion oder ähnliche technische Geräte, genutzt werden kann. Dadurch lassen sich erhebliche Kosten einsparen und der Platzbedarf der vom Patienten mitzuführen Messgeräte wird erheblich reduziert. Insbesondere kann das mobile Datenübertragungsgerät, insbesondere das Mobiltelefon, derart ausgestaltet sein, dass die Kamera eine Digitalkamera aufweist.

Die Haltevorrichtung kann z. B. derart ausgestaltet sein, dass die Verbindungseinrichtung eine Aufklemmschiene zum Aufklemmen der Haltevorrichtung auf das mobile Datenübertragungsgerät, insbesondere auf das Mobiltelefon, aufweist. Alternativ oder zusätzlich kann die Haltevorrichtung auch eine Auflagevorrichtung zum positionierten, d. h. insbesondere definierten Auflegen des mobilen Datenübertragungsgerätes auf die Haltevorrichtung, oder zum positionierten Auflegen der Haltevorrichtung auf das mobile Datenübertragungsgerät aufweisen. Beispielsweise kann diese Auflagevorrichtung eine einfache Auflagefläche umfassen, welche durch einfaches Auflegen beispielsweise des mobilen Datenübertragungsgerätes auf die Auflagefläche eine definierte Positionierung der Haltevorrichtung relativ zum mobilen Datenübertragungsgerät ermöglicht.

Weiterhin kann die Haltevorrichtung zusätzlich eine Positioniervorrichtung zur Veränderung der räumlichen Ausrichtung und/oder relativen Anordnung des mindestens einen Testelements relativ zu der mindestens einen Kamera aufweisen. Auf diese Weise lässt sich beispielsweise das mindestens eine Testelement optimal relativ zur Kamera positionieren, oder es können nacheinander mehrere Bereiche des Testelements vom Sichtbereich der mindestens einen Kamera erfasst werden. Diese Positioniervorrichtung kann beispielsweise so ausgestaltet sein, dass eine manuelle Positionierung durch den Patienten oder auch eine automatische Positionierung (beispielsweise durch einen entsprechenden Antrieb, zum Beispiel einen computergesteuerten Antrieb) erfolgen kann. Auch eine manuelle Positionierung, beispielsweise mittels einer Handkurbel, ist denkbar. Die Verbindungseinrichtung, insbesondere die Aufklemmschiene, sowie die Positioniervorrichtung können insbesondere auf eine Auswahl von kommerziell verfügbaren mobilen Datenübertragungsgeräten, insbesondere auf eine Auswahl bestimmter Mobiltelefone mit Kamerafunktion, angepasst sein. So kann ein Benutzer beispielsweise kostengünstig eine geeignete Haltevorrichtung für einen entsprechenden Typ von Mobiltelefon erwerben.

Auch kann die Halterung zusätzlich mindestens ein optisches Element aufweisen, beispielsweise mindestens ein Linsenelement und/oder mindestens ein Spiegelelement. Dadurch kann die Halterung auf die optischen Eigenschaften eines bestimmten Kameratyps, beispielsweise eines in einer bestimmten Mobiltelefonserie verwendeten Kameratyps angepasst werden. Auf diese Weise lässt sich insbesondere der Bauraum der Halterung bei vorgegebener Brennweite der Kamera reduzieren.

In einer vorteilhaften Ausgestaltung der Erfindung weist die Haltevorrichtung zusätzlich mindestens eine Lichtquelle zur Beleuchtung mindestens eines Teilbereichs des mindestens einen Testelements auf. Insbesondere kann es sich dabei um eine Lichtquelle zur Emission von sichtbarem und/oder infrarotem und/oder ultraviolettem Licht handeln. Diese Lichtquelle kann beispielsweise durch eine in die Haltevorrichtung eingebrachte Energiequelle, beispielsweise eine Batterie oder einen Akkumulator, gespeist werden, oder es kann auch eine Schnittstelle zum mobilen Datenübertragungsgerät vorgesehen sein, so dass die Lichtquelle elektrische Energie (z, B. über ein entsprechendes Kabel) zumindest teilweise von der elektrischen Energieversorgung des mobilen Datenübertragungsgerätes bezieht.

Diese Weiterbildung der Erfindung hat den Vorteil, dass eine Auswertung des Testelements mittels der mindestens einen Kamera auch unter ungünstigen Umgebungslichtverhältnissen erfolgen kann. So kann insbesondere die Haltevorrichtung derart ausgestaltet sein, dass das Testelement vollständig vom Umgebungslicht abgeschirmt wird und somit vollständig unabhängig vom Umgebungslicht arbeitet. Die Wellenlänge bzw. die Wellenlängen, welche von der mindestens einen Lichtquelle emittiert werden, sind dabei auf die Ausgestaltung des Testelements anzupassen. Bewirkt beispielsweise die oben beschriebene Reaktion zwischen einem Reagenz und der nachzuweisenden chemischen Substanz einer Farbveränderung im sichtbaren Bereich, so bietet sich insbesondere eine Lichtquelle an, welche sichtbares Licht emittiert. Es sind jedoch auch andere Wellenlängen möglich. Ultraviolettes Licht bietet sich insbesondere dann an, wenn Fluoreszenznachweise geführt werden. Beispielsweise kann das Testelement derart ausgestaltet sein, dass ein bestimmtes Reagenz des Testelements bei Reaktion mit einer nachzuweisenden chemischen Substanz zu fluoreszieren beginnt, oder dass eine entsprechende Fluoreszenz durch die Reaktion mit einer nachzuweisenden chemischen Substanz gelöscht wird (Quenching).

Das erfindungsgemäße System zur Auswertung eines Testelements, insbesondere eines Testelements zur medizinischen oder chemischen Analyse, weist also, wie oben beschrieben, mindestens ein entsprechendes Testelement mit mindestens einem optischen Testfeld und mindestens ein mobiles Datenübertragungsgerät, insbesondere ein Mobiltelefon, mit mindestens einer Kamera mit einem Sichtbereich auf. Optional kann das System auch mindestens eine Haltevorrichtung entsprechend einer der beschriebenen Ausgestaltungen aufweisen.

Mittels eines derartigen erfindungsgemäßen Systems kann beispielsweise ein im Folgenden beschriebenes erfindungsgemäßes Verfahren zur Auswertung eines Testelements durchgeführt werden. Die dargestellten Verfahrensschritte sind nicht notwendigerweise in der dargestellten Reihenfolge durchzuführen, und es können auch zusätzliche, nicht dargestellte Verfahrensschritte durchgeführt werden. Weiterhin können einzelne Verfahrensschritte auch zeitgleich oder zeitlich überlappend durchgeführt werden, oder es kann eine Wiederholung einzelner oder mehrerer Verfahrensschritte erfolgen.

In einem ersten Verfahrensschritt wird mindestens eine Probe, vorzugsweise eine flüssige Probe, insbesondere eine Probe welche Blut und/oder Blutbestandteile und/oder Speichel und/oder Urin aufweist, mit mindestens einem Testelement, (wobei es sich insbesondere um eine Testelement der oben beschriebenen Art handeln kann) in Kontakt gebracht.

In einem zweiten Verfahrensschritt wird mittels der mindestens einen Kamera des mobilen Datenübertragungsgerätes mindestens eine Bildinformation aufgenommen. Dabei kann es sich insbesondere um digitale Bilddaten handeln. Diese digitalen Bilddaten können insbesondere in einem Datenspeicher des mobilen Datenübertragungsgerätes, insbesondere des Mobiltelefons, abgespeichert werden.

Das Verfahren kann dahingehend erweitert werden, dass das mindestens eine Testelement in eine erfindungsgemäße Haltevorrichtung eingebracht wird. Wie oben beschrieben, kann dabei die Reihenfolge des Einbringens des Testelements in die Haltevorrichtung und das Applizieren der Probe zeitlich vertauscht sein, so dass beispielsweise auch zuerst ein Einbringen des Testelements in die Haltevorrichtung und anschließend ein Applizieren der Probe erfolgen kann.

Weiterhin kann das mobile Datenübertragungsgerät mit der mindestens einen Haltevorrichtung verbunden werden, wobei die Verbindung dergestalt erfolgt, dass der Sichtbereich das mindestens eine optische Testfeld (bzw. mindestens eins der optischen Testfelder) des mindestens einen Testelements erfasst. Wiederum kann auch dieser Verfahrensschritt beispielsweise vor oder zwischen den bereits beschriebenen Verfahrensschritten erfolgen. Wie oben beschreiben, kann der Sichtbereich dabei auch mehrere optische Testfelder gleichzeitig erfassen, oder es können auch mehrere Testelemente gleichzeitig von einer Kamera erfasst werden.

Die mindestens eine Bildinformation kann anschließend optional in einem Skalierungsschritt reskaliert werden. Dazu kann beispielsweise die Bildinformation ausgewertet werden, wobei z. B. mittels eines Bildverarbeitungsalgorithmus automatisch eine Skalierungsreferenz (z. B. eine Längenskala oder ein Barcode) auf dem Testelement erkannt wird. Anhand beispielsweise einer bekannten Dimension dieser Skalierungsreferenz kann auf die tatsächliche Größe des Bildausschnitts geschlossen werden, welcher durch die mindestens eine Bildinformation erfasst ist. Anschließend kann ein Skalierungsfaktor bestimmt werden und die Bildinformation beispielsweise auf eine Normgröße (entsprechend beispielsweise einem Norm-Bildausschnitt) reskaliert werden. Auf diese Weise kann das Verfahren auch mit einem nicht bekannten Abstand zwischen Kamera und Testelement durchgeführt werden, beispielsweise wenn das mobile Datenübertragungsgerät freihändig von einem Benutzer gehalten wird, ohne dass eine Haltevorrichtung verwendet wird. Im Folgenden wird zwischen den originären Bildinformationen und reskalierten Bildinformationen begrifflich nicht unterschieden.

Weiterhin kann anschließend in einem weiteren Verfahrensschritt die aufgenommene Bildinformation mittels eines ersten Auswertungsverfahrens in dem mobilen Datenübertragungsgerät analysiert werden, wobei mindestens ein Auswertungsergebnis erzeugt wird. Insbesondere kann durch Auswertung einer Farbinformation des mindestens eines optischen Testfeldes, beispielsweise einer Schwärzung, einer Farbveränderung, einer Reflektionsveränderung oder einer Fluoreszenzveränderung, auf die Anwesenheit einer oder mehrerer vorgegebener chemischer Substanzen geschlossen werden, oder es können alternativ oder zusätzlich auch die Konzentration bzw, Konzentrationen dieser vorgegebenen chemischen Substanzen bestimmt werden. Somit kann das mindestens eine Auswertungsergebnis beispielsweise eine Analytkonzentration beinhalten. Derartige Auswertungsverfahren sind (z. B. aus dem eingangs genannten Stand der Technik) bekannt.

Das mindestens eine Auswertungsergebnis kann anschließend ausgegeben werden. Die Ausgabe kann beispielsweise über ein Display, insbesondere ein Display eines Mobiltelefons, erfolgen. Die Ausgabe kann beispielsweise in Form eines einfachen Zahlenwertes, mehrerer Zahlenwerte, einer oder mehrerer Tabellen oder auch in Fonn einer graphischen Aufarbeitung erfolgen, Insbesondere kann das mobile Datenübertragungsgerät auch eine Datenbankfunktion aufweisen, beispielsweise eine Datenbankfunktion, in welcher Auswertungsergebnisse über einen bestimmten Zeitraum verwaltet werden. So kann beispielsweise die Blutglukosekonzentration als Funktion der Zeit dargestellt werden, so dass ein Patient einen Überblick über Schwankungen und kurzfristige oder langfristige Entwicklungen der Slutglukosekonzentration erhält. Neben oder alternativ zu einer Blutglukosekonzentrationsberechnung ist dabei auch eine Berechnung der Konzentration von beispielsweise Cholesterin, Alkohol, Kreatinin und/oder Ketonen sowie von anderen chemischen Substanzen möglich.

Neben einer Ausgabe in Form visueller Daten ist auch eine Ausgabe in anderer Form möglich. So ist beispielsweise eine Ausgabe in elektronischer Form an einen weiteren Computer, einen Drucker oder an einen akustischen Signalgeber möglich.

Das erfindungsgemäße Verfahren kann insbesondere auch wiederholt durchgeführt werden. So kann insbesondere das Verfahren derart ausgestaltet sein, dass der Verfahrensschritt der Aufnahme mindestens einer Bildinformation mittels der mindestens einen Kamera wiederholt durchgeführt wird. Auf diese Weise lässt sich beispielsweise auch die zeitliche Entwicklung des mindestens einen optischen Testfeldes beobachten. So kann beispielsweise in regelmäßigen Abständen ein Bild aufgenommen werden. Auf diese Weise lassen sich aus der zeitlichen Entwicklung zusätzliche Informationen über die Zusammensetzung beispielsweise flüssiger Proben gewinnen. Auch wird das beschriebene erfindungsgemäße System auf diese Weise unabhängiger gegenüber Variationen des zeitlichen Abstandes zwischen Applikation der Probe auf das Testelement und Auswertung des Testelements.

Das oben beschriebene erfindungsgemäße System lässt sich weiterhin dahingehend erweitern, dass das System weiterhin mindestens einen Auswertungsserver aufweist. Der mindestens eine Auswertungsserver soll über Mittel zum Empfangen mindestens einer Information, wobei die mindestens eine Information vorzugsweise die mindestens eine Bildinformation umfasst, von dem mindestens einen mobilen Datenübertragungsgerät verfügen. Dabei sei darauf hingewiesen, dass hier und im Folgenden unter "Mitteln" geeignete Hardware (z. B. Prozessoren, Speicher etc.) und/oder geeignete Software vezstanden werden kann. Beispielsweise kann es sich bei den Mitteln zum Empfangen der mindestens einen Bildinformation um einen Anschluss des mindestens einen Auswertungsservers an ein Netzwerk, beispielsweise Internet oder ein LAN-Netzwerk, handeln, sowie um die entsprechende Datenübertragungssoftware. Auch andere Mittel zum Empfangen von Bildinformationen sind denkbar, beispielsweise die Einbindung des mindestens einen Auswertungsservers in ein Mobilfunknetz. Neben Bildinformationen können dabei auch weitere Informationen übertragen werden. So kann die mindestens eine Information beispielsweise das oben beschriebene mindestens eine Auswertungsergebnis umfassen. Weiterhin kann die mindestens eine Information Informationen über den Benutzer des Systems (zum Beispiel Patientendaten) oder Informationen über die verwendeten Testelemente umfassen.

Weiterhin soll der mindestens eine Auswertungsserver Mittel zum Analysieren der mindestens einen Information, insbesondere Mittel zum Berechnen einer Konzentration mindestens einer vorgegebenen chemischen Substanz, aufweisen. Diese Mittel zum Analysieren der mindestens einen Information, vorzugsweise der mindestens einen Bildinformation, können insbesondere einen Computer oder ein Computersystem umfassen.

Weiterhin soll der mindestens eine Auswertungsserver Mittel zum Übersenden mindestens eines Analysenergebnisses an das mindestens eine mobile Datenübertragungsgerät des erfindungsgemäßen Systems umfassen. Insbesondere kann es sich dabei wiederum um einen Anschluss an ein Netzwerk und/oder eine Einbindung des mindestens einen Auswertungsservers in ein Telekommunikatioztsnetz, vorzugsweise in ein Mobilfunknetz, handeln.

Mittels dieser vorteilhaften Ausgestaltung des erfindungsgemäßen Systems lässt sich das oben beschriebene Verfahren erfindungsgemäß dahingehend erweitern, dass die mindestens eine Information, insbesondere die mindestens eine mittels der mindestens einen Kamera aufgenommene Bildinformation, von dem mobilen Datenübertragungsgerät an den mindestens einen Auswertungsserver übermittelt wird. Dabei kann die Bildinformation vollständig oder auch nur teilweise übermittelt werden, beispielsweise kann die mittels der einen Kamera aufgenommene Bildinformation zunächst vorverarbeitet werden, um anschließend an den mindestens einen Auswertungsserver übermittelt zu werden. Auf diese Weise lassen sich beispielsweise Datenströme minimieren, um eine optimale Ressourcenausnutzung zu erreichen und den Zeitbedarf für die Übermittlung der Daten zu minimieren. Insbesondere können zusätzlich zu der mindestens einen Bildinformation auch zusätzliche Informationen übertragen werden, beispielsweise Informationen über den Patienten oder über die Art der verwendeten Testelemente.

Auf dem mindestens einen Auswertungsserver kann anschließend die mindestens eine Bildinformation und gegebenenfalls Zusatzinformationen mittels eines zweiten Auswertungsverfahrens von dem mindestens einen Auswertungsserver analysiert werden. Anschließend kann ein dabei ermitteltes Analyseergebnis von dem mindestens einen Auswertungsserver an das mobile Datenübertragungsgerät übermittelt werden.

Das Verfahren kann also derart ausgestaltet werden, dass eine Auswertung der Bildinformationen entweder auf dem mobilen Datenübertragungsgerät selbst erfolgt, oder auf dem mindestens einen Auswertungsserver. Alternativ kann die Auswertung der Bildinformationen auch geteilt werden, beispielsweise indem eine Vorverarbeitung bereits auf dem mobi-Ien Datenübertragungsgerät stattfindet und eine weitere (z. B. exaktere) Verarbeitung auf dem mindestens einen Auswertungsserver. Zusätzliche Verfahrensschritte können auf dem mindestens einen Auswertungsserver durchgeführt werden, beispielsweise können die A-nalyseergebnisse in einer Datenbank gespeichert werden, um für eine spätere Auswertung zur Verfügung zu stehen. Weiterhin besteht auch die Möglichkeit, von dem mindestens einen Auswertungsserver Daten an einen behandelnden Arzt oder an ein Krankenhaus zu übermitteln, so dass beispielsweise eine Dauerüberwachung eines Patienten stattfinden kann. Diese Ausgestaltung der Erfindung trägt also dazu bei, den so genannten "Home-Care"-Gedanken, welcher in der modernen Medizin immer mehr Einzug hält, zu fördem und dadurch beispielsweise eine Aufnahme des Patienten in ein Krankenhaus in vielen Fällen überflüssig zu machen. Dadurch werden enorme Kosten im Gesundheitswesen gespart.

Das erfindungsgemäße Verfahren kann insbesondere dahingehend weitergebildet werden, dass die Übermittlung des Analyseergebnisses von dem mindestens einen Auswertungsserver an das mobile Datenübertragungsgerät drahtlos erfolgt, insbesondere über ein Mobilfunknetz. Besonderes vorteilhaft ist es, wenn die Datenübermittlung über den so genannten Short-Message-Service (SMS) erfolgt, da auf diese Weise Mobilfunkressourcen optimal ausgenutzt werden und Kommunikationskanäle geschont werden. So kann beispielsweise der Patient eine SMS mit einem einfachen Analyseergebnis, beispielsweise einer Blutglukosekonzentration, erhalten. Auf diese Weise kann die Auswertung des Testelements nahezu vollständig auf den mindestens einen Auswertungsserver übertragen werden, wodurch die Ressourcen des mobilen Datenübertragungsgerätes, insbesondere des Mobiltelefons, geschont werden.

Weiterhin kann das erfindungsgemäße System dahingehend weitergebildet werden, dass der mindestens eine Auswertungsserver zusätzlich Mittel zum Authentifizieren eines Benutzers aufweist. Das beschriebene erfindungsgemäße Verfahren kann dann beispielsweise derart ausgestaltet sein, dass gemeinsam mit der mindestens einen Bildinformation benutzerspezifische Informationen, beispielsweise eine Identifikationsnummer, und eventuell ein Passwort mit an den Auswertungsserver übermittelt werden. Diese Übermittlung kann beispielsweise automatisch erfolgen oder kann auch eine Eingabe durch den Benutzer erfordern. Auf diese Weise kann ein Benutzer beispielsweise Zugriff auf Datenbankinformationen exhalten, wobei sichergestellt ist, dass nur ein berechtigter Benutzer Zugriff auf seine entsprechenden Daten hat. Auch kann beispielsweise automatisch eine Gerätenummer an den mindestens einen Auswertungsserver übermittelt werden, so dass beispielsweise der Auswertungsserver automatisch das mindestens eine mobile Datenübertragungsgerät erkennt. Beispielsweise kann der mindestens eine Auswertungsserver die Gerätenummer mit einer entsprechenden Liste von Nummern vergleichen und so den jeweiligen Nutzer ermitteln. Auch entsprechende Zahlungsmodalitäten können auf diese Weise geregelt werden, beispielsweise indem bei jeder Benutzung des Systems durch einen Patienten ein gewisser Betrag von einem Konto abgebucht wird oder eine entsprechende Rechnung um einen bestimmten Betrag erhöht wird. So kann beispielsweise in regelmäßigen Zeitintovallen ein Nutzer eine entsprechende automatische Rechnung für die Nutzung des Verfahrens und des Auswertungsservers erhalten.

Weiterhin kann das beschriebene System dahingehend erweitert werden, dass ein erfindungsgemäßes Kalibrierelement eingesetzt wird. Dieses Kalibrierelement dient zum Kalibrieren des erfindungsgemäßen Systems gemäß einer der oben beschriebenen Ausgestaltungen. Das Kalibrierelement entspricht vorteilhafterweise in seinen äußeren Abmessungen im Wesentlichen dem mindestens einen Testelement, welches in dem erfindungs gemäßen System eingesetzt wird. Insbesondere ist dadurch gewährleistet, dass das Kalibrierelement in die mindestens eine Halterung der erfindungsgemäßen Haltevorrichtung eingesetzt werden kann. Alternativ kann das Kalibrierelement jedoch auch ohne die Haltevorrichtung eingesetzt werden.

Das Kalibrierelement weist mindestens ein optisches Kalibrierfeld auf, wobei das mindestens eine optische Kalibrierfeld mindestens ein optisches Signal erzeugt. Bei diesem optischen Signal kann es sich wiederum, wie oben im Zusammenhang mit dem mindestens einen Testelement beschrieben, beispielsweise um ein Kalibrierfarbfeld und/oder ein Kalibrierfluoreszenzield handeln. Zusätzlich kann auf dem Kalibrierelement auf noch, analog zu dem oben beschriebenen Testelement, eine Referenzeinrichtung zur Erzeugung der -mindestens einen Referenz, insbesondere einer Farbreferenz und/oder einer Fluoreszenzreferenz und/oder einer Positionsreferenz und/oder einer Skalierungsreferenz, angeordnet sein. Alternativ oder zusätzlich zur Verwendung eines separaten Kalibrierelements können auch ein oder mehrere Referenzeinrichtungen unmittelbar an oder auf der Haltevorrichtung angebracht sein und für eine entsprechende Kalibrierung verwendet werden.

Das erfindungsgemäße Kalibrierelement in einer der beschriebenen Ausgestaltungen kann in einer Weiterbildung des erfindungsgemäßen Verfahrens dahingehend genutzt werden, dass vor der Auswertung des Testelements zunächst mittels der mindestens einen Kamera mindestens eine Kalibrier-Bildinformation aufgenommen wird. Wenn das Kalibrierelement bekannte Eigenschaften aufweist, so kann auf diese Weise das erfindungsgemäße System kalibriert werden.

Diese Kalibration kann insbesondere dazu benutzt werden, um unterschiedliche Hardeware-Eigenschaften, beispielsweise unterschiedliche Eigenschaften einer Kamera eines Mobiltelefons, auszugleichen. Auch unterschiedliche Eigenschaften der eingesetzten Testelemente lassen sich ausgleichen. So kann beispielsweise jeder Charge von Testelementen (beispielsweise einer neuen Packung Testelemente) ein oder mehrere dieser Kalibrierelemente beigefügt sein. Der eigentliche Kalibriervorgang kann beispielsweise auf dem mobilen Datenübertragungsgerät selbst oder auch auf dem mindestens einen Auswertungsserver erfolgen. In letzterem Fall werden beispielsweise von dem mindestens einen mobilen Datenübertragungsgerät an den mindestens einen Auswertungsserver neben den Bildinformationen vorteilhafterweise auch Kalibrierinformationen übersandt.

Weiterhin gehört zum Umfang der Erfindung auch ein Computerprogramm, das bei Ablauf auf einem Computer oder Computernetzwerk eines mobilen Datenübertragungsgerätes das erfindungsgemäße Verfahren in einer seiner Ausgestaltungen teilweise durchführt. Insbesondere kann die beschriebene Aufnahme der mindestens einen Bildinformation, die Analyse der mindestens einen Bildinformation, die Aufnahme einer Kalibrier-Bildinformation, die Reskalierung und/oder die Übermittlung der mindestens einen Bildinformation von dem mobilen Datenübertragungsgerät an dem mindestens einen Auswertungsserver durch das Computerprogramm gesteuert erfolgen. Insbesondere kann das Computerprogramm Programmcode-Mittel aufweisen, welche auf einem computerlesbaren Datenträger gespeichert sind.

Weiterhin gehört zum Umfang der Erfindung ein Datenträger, auf dem eine Datenstruktur gespeichert ist, die nach einem Laden in einen Arbeits- und/oder Hauptspeicher eines Computers oder Computernetzwerkes eines mobilen Datenübertragungsgerät die genannten Verfahrensschritte des erfindungsgemäßen Verfahrens in einer seiner Ausgestaltungen ausführen kann. Auch ein entsprechendes Computerprogramm-Produkt mit auf einem maschinenlesbaren Träger gespeicherten Programmcode-Mitteln wird erfindungsgemäß beansprucht, Dabei wird unter einem Computerprogramm-Produkt das Programm als handelbares Produkt verstanden. Es kann grundsätzlich in beliebiger Form vorliegen, so zum Beispiel auf Papier oder einem computerlesbaren Datenträger und kann insbesondere über ein Datenüberiragungsnetz verteilt werden.

- Weiterhin wird auch ein entsprechendes Computerprogramm mit Programmcode-Mitteln, welche insbesondere auf einem computerlesbaren Datenträger gespeichert sein können, sowie ein entsprechendes Computer-Programmprodukt beansprucht, welches die Ausführung der Verfahrensschritte des erfindungsgemäßen Verfahrens in einer seiner Ausgestaltungen, welche von dem mindestens einen Auswertungsserver durchgeführt werden, unterstützt. Dies sind insbesondere die oben beschriebenen Verfahrensschritte der Analyse der mindestens einen Bildinformation und der Übermittlung eines Analyseergebnisses von dem mindestens einen Auswertungsserver an das mobile Datenübertragungsgerät.

Weitere Einzelheiten und Merkmale der Erfindung ergeben sich aus der nachfolgenden Beschreibung von bevorzugten Ausführungsbeispielen in Verbindung mit den Unteransprüchen, Hierbei können die jeweiligen Merkmale für sich alleine oder zu mehreren in Kombination miteinander verwirklicht sein. Die Erfindung ist nicht auf die Ausfuhrungsbeispiele beschränkt. Die Ausfuhrungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigt:
- Figur 1: ein erstes Ausführmigsbeispiel eines Systems zur Auswertung eines Testelements mit einem ersten Ausführungsbeispiel einer Haltevorrichtung und einem Mobiltelefon;
- Figur 1A: ein zweites Ausführungsbeispiel einer Haltevorrichtung mit einem bandförmigen Testelement;
- Figur 1B: ein zweites Ausführungsbeispiel eines Systems zur Auswertung eines Testelements mit der Haltevorrichtung gem. Figur 1A und einem Mobiltelefon in Seitenansicht;
- Figur 1C: ein drittes Ausführungsbeispiel eines Systems zur Auswertung eines Testelements mit einem Mobiltelefon und einer dritten Ausführungsform einer Haltevorrichtung in Seitenansicht;
- Figur 2: ein erstes Ausführungsbeispiel eines Testelements mit optischen Testfeldern;
- Figur 2A: ein zweites Ausführungsbeispiel eines Testelements;
- Figur 3: ein Ausführungsbeispiel eines Kalibrierelements zum Einsatz in einem System zur Auswertung eines Testelements;
- Figur 4: ein viertes Ausführungsbeispiel eines Systems zur Auswertung eines Testelements unter Verwendung eines Mobilfunknetzes und eines Auswertungsservers;
- Figur 5: einen schematischen Ablaufplan eines Verfahrens zum Ausweiten eines Testelements; und
- Figur 6: einen Ausschnitt eines Ablaufplans eines Verfahrens zur Auswertung eines Testelements unter Einschluss eines Kalibriervorgangs.

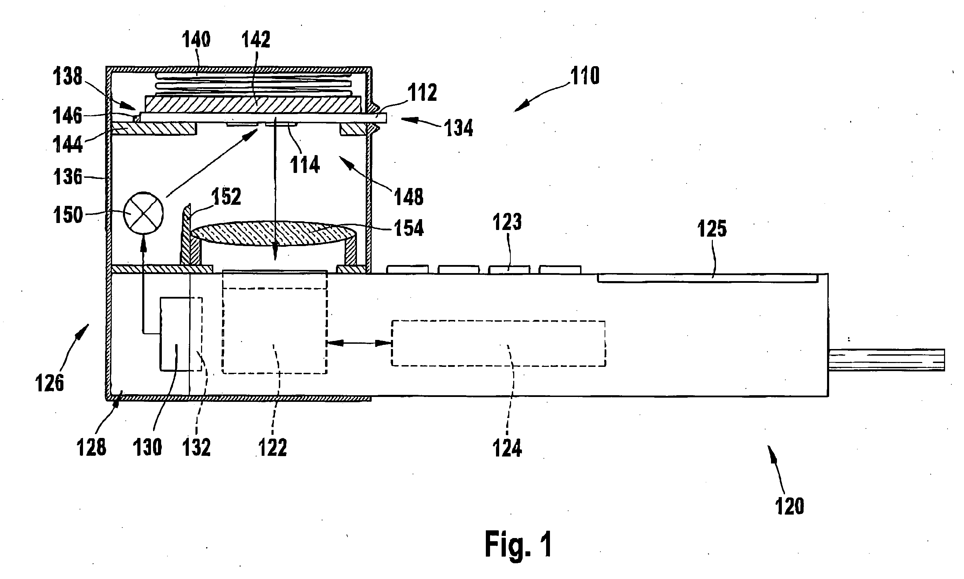

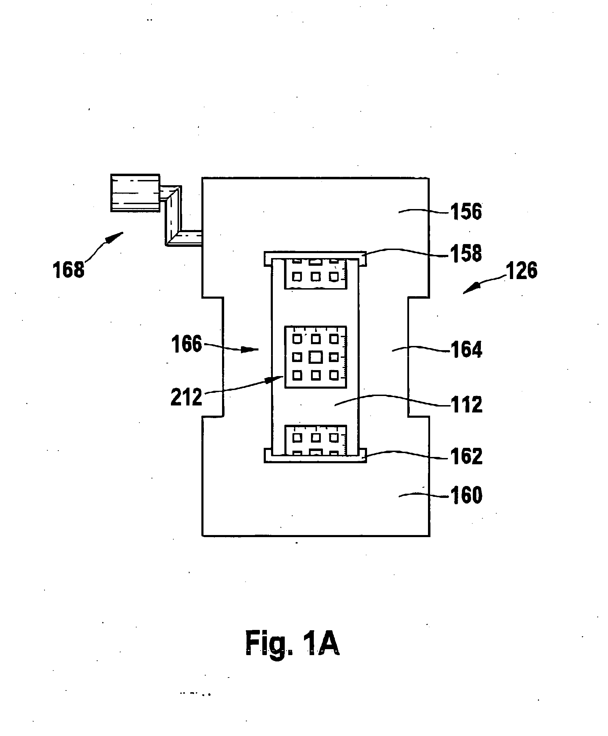

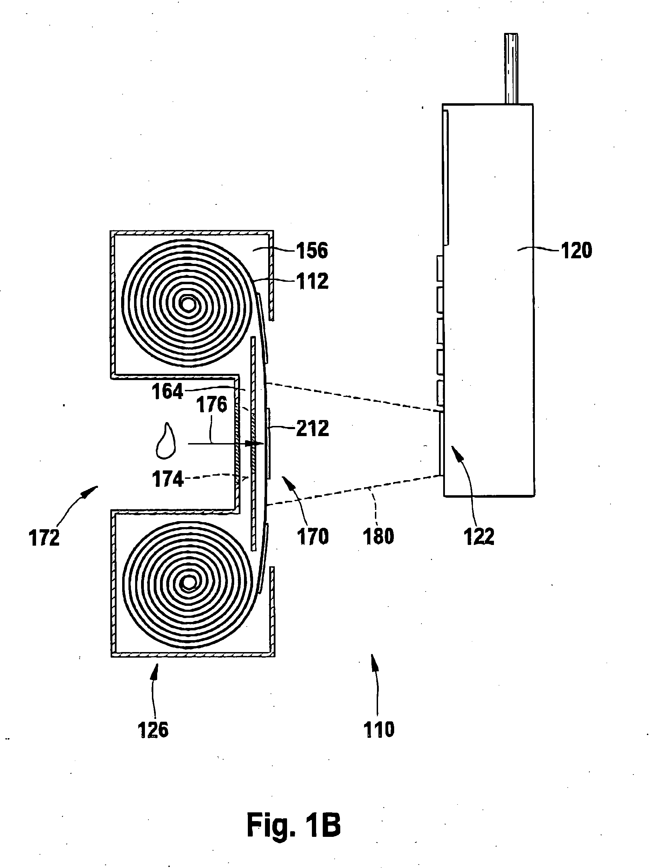

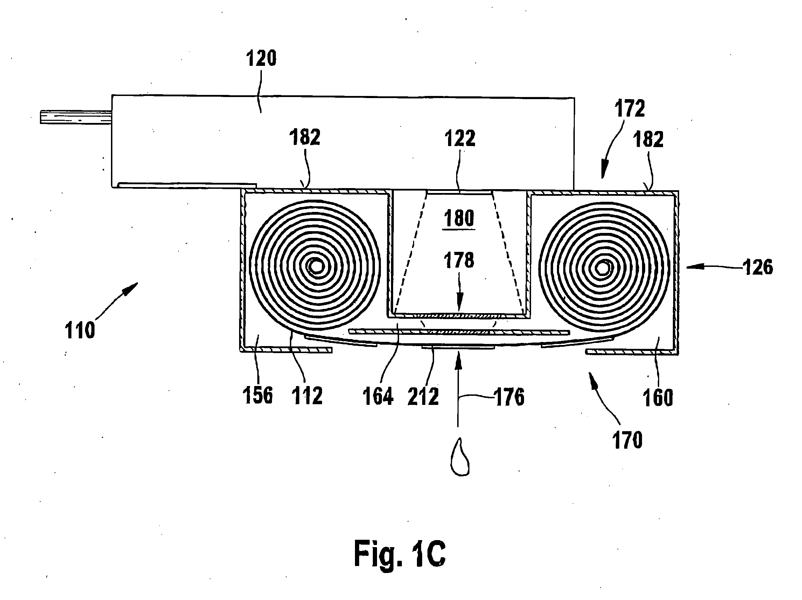

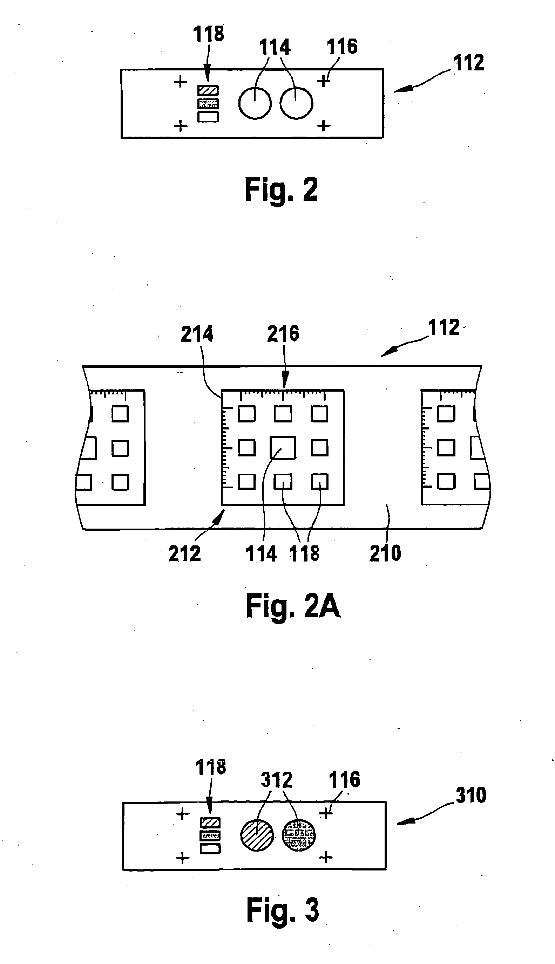

In Figur 1 ist ein einfaches Ausführungsbeispiel eines erfindungsgemäßen Systems 110 zur Auswertung eines Testelements 112 dargestellt. In diesem Ausführungsbeispiel handelt es sich bei dem Testelement 112 beispielsweise um einen Teststreifen 112 gemäß dem Ausführungsbeispiel in Figur 2. Der Teststreifen 112 weist in diesem Ausführungsbeispiel zwei optische Testfelder 114, innerhalb welcher auf einem Trägermaterial selektive Reagenzien zum Nachweis chemischer Substanzen, beispielsweise Blutglukose, aufgebracht sind. Derartige optische Testfelder sind aus dem Stand der Technik bekannt und beispielsweise in den oben beschriebenen Druckschriften offenbart. Neben den optischen Testfeldem 114 weist der Teststreifen 112 Positionsmarken 116 auf sowie Referenzfelder 118 mit unbekannten Färbungen oder Graustufen.

Das System 110 gemäß dem in Figur 1 dargestellten Ausführungsbeispiel weist neben dem Teststreifen 112 im Wesentlichen zwei Komponenten auf: ein Mobiltelefon 120 mit einer Kamera 122, einem Mikroprozessor 124, einer Tastatur 123 und einem Display 125 sowie eine Haltevorrichtung 126. Die Haltevorrichtung 126 weist ihrerseits eine Aufklemmschiene 128 auf, mittels derer die Haltevorrichtung 126 auf das Mobiltelefon 120 aufgesteckt werden kann, wie in Figur 1 dargestellt. In diesem Ausführungsbeispiel ist die Aufklemmschiene 128 mit einer Schnittstelle 130 versehen, welche beim Aufstecken der Haltevorrichtung 126 auf das Mobiltelefon 120 mit einer entsprechenden Schnittstelle 132 des Mobiltelefons 120 verbunden wird.

Weiterhin weist die Haltevorrichtung 126 in diesem Ausführungsbeispiel einen Einschubschlitz 134 in einem Gehäuse 136 der Haltevorrichtung 126 auf, durch welchen der Teststreifen 112 in die Haltevorrichtung 126 eingeschoben werden kann. Innerhalb der Haltevorrichtung 126 ist der Teststreifen 112 dann in einer Teststreifenaufnahme 138 gehaltert, wobei der Teststreifen 112 mittels einer Andruckfeder 140 und einem Andruckstempel 142 gegen eine Halterung 144 gedrückt wird, Ein Anschlag 146 verhindert dabei, dass der Teststreifen 112 weiter in die Teststreifenaufnahme 138 hinein geschoben werden kann, so dass beispielsweise verhindert wird, dass der Teststreifen 112 vollständig in die Haltevorrichtung 126 hineingleiten kann und somit für einen Benutzer nicht mehr greifbar ist.

Die Halterung 144 weist eine Aussparung 148 auf. Der Teststreifen 112 wird derart in die Teststreifenaufnahme 138 eingeschoben, dass die optischen Testfelder 114 auf der der Kamera 122 zugewandten Seite des Teststreifens 112 zu liegen kommen. Die Aussparung 148 ist dabei so dimensioniert, dass sowohl die optischen Testfelder 114 als auch die Positionsmarken 116 und die Referenzfelder 118 innerhalb der Aussparung 148 zu liegen kommen.

Weiterhin weist die Haltevorrichtung 126 eine Lichtquelle 150 auf, welche beispielsweise mit einer zusätzlichen Optik versehen sein kann und welche die optischen Testfelder 114 und gegebenenfalls die Positionsmarken 116 und die Referenzfelder 118 entsprechend beleuchtet. Durch eine Streulichtblende 152 wird verhindert, dass Licht von der Lichtquelle 150 direkt in die Kamera 122 gelangen kann. Das Gehäuse 136 der Haltevorrichtung 126 ist ansonsten bei auf das Mobiltelefon 120 aufgesteckter Haltevorrichtung 126, weitgehend lichtdicht ausgestaltet, so dass kein Umgebungslicht die Messung negativ beeinflussen kann.

Weiterhin weist die Haltevorrichtung 126 eine Linse 154 auf, welche bewirkt, dass die optischen Testfelder 114 mit ausreichender Qualität von der Kamera 122 abgebildet werden können. Diese Linse 154 kann beispielsweise als Makrolinse 154 ausgestaltet sein, um eine Abbildung eines nahe vor der Kamera 122 positionierten Objektes zu ermöglichen.

In dem Ausführungsbeispiel gemäß Figur 1 kann beispielsweise die Lichtquelle 150 und somit die Aufnahme von Bildern der optischen Testfelder 114 durch das Mobiltelefon 120 gesteuert werden, insbesondere durch den Mikroprozessor 124. Diese Steuerung kann insbesondere über die Schnittstellen 130 und 132 erfolgen. So kann beispielsweise die Lichtquelle 150 über die Schnittstellen 130, 132 vom Mobiltelefon 120 mit elektrischer Energie versorgt werden. Alternativ oder zusätzlich kann die Haltevorrichtung 126 auch eine eigene Energiequelle aufweisen. Insbesondere kann die Lichtquelle 150 auch gepulst betrieben werden, wobei beispielsweise die Lichtpulse über die Schnittstellen 130, 132 mit der Aufnahmefunktion der Kamera 122 zeitlich koordiniert (insbesondere vom Mikroprozessor 124 getriggert) werden.

In dem Ausführungsbeispiel gemäß Figur 1 erfolgt die Applikation einer Probe auf den Teststreifen 112 außerhalb der Haltevorrichtung 126, beispielsweise durch Abstreifen eines Blutstropfens. Dies bedeutet, dass ein Patient zunächst eine Probe appliziert (beispielsweise einen Blutstropfen) und anschließend den Teststreifen 112 durch den Einschubschlitz 134 in die Teststreifenaufnahme 138 einschiebt. Wie oben bereits beschrieben, lassen sich jedoch auch andere Ausführungsbeispiele realisieren, beispielsweise unter Verwendung von Magazinsystemen, bei denen mehrere Teststreifen 112 innerhalb der Haltevorrichtung 126 gelagert sind, und/oder unter Verwendung eines Kapillarsystems,

Die Andruckfeder 140, der Andruckstempel 142, die Halterung 144 und der Anschlag 146 wirken in diesem Ausführungsbeispiel, als einfache Positionierungsvorrichtung. Alternativ kann jedoch auch eine komplexere Positionierungsvorrichtung eingesetzt werden, beispielsweise in Form von Manipulatoren, mittels derer sich der Teststreifen 112 innerhalb der Haltevorrichtung 126 relativ zur Kamera 122 verschieben und/oder ausrichten lässt.

Weiterhin ist in diesem Ausfubrungsbeispiel die Linse 154 räumlich fixiert gelagert. Alternativ lässt sich jedoch auch eine Ausführung realisieren, bei der die Linse 154 beweglich gelagert ist, um beispielsweise automatisch oder manuell verschoben zu werden, beispielsweise zur Durchführung eines Fokussierungsschrittes. Dieser Fokussierungsschritt kann insbesondere die optische Qualität einer Abbildung stark erhöhen.

In Figur 3 ist ein Kalibrierelement 310 in Form eines Teststreifens dargestellt. Dieses Kalibrierelement 310 weist die gleichen Abmessungen wie der in Figur 2 dargestellte Teststreifen 112 auf. Wiederum verfügt das Kalibrierelement 310 über die Positionsmarken 116 und Referenzfelder 118, Anstelle der optischen Testfelder 114 sind jedoch bei dem Kalibrierelement 310 optische Kalibrierfelder 312 vorgesehen, welche an den entsprechenden Stellen auf dem Kalibrierelement 310 angeordnet sind. Diese optischen Kalibrierfelder 312 weisen bekannte Färbungen, bekannte Reflexionseigenschaften und/oder bekannte Graustufe auf.

In Figur 1A ist ein zweites, zur Haltevorrichtung 126 in Figur 1 alternatives Ausführungsbeispiel einer erfindungsgemäßen Haltevorrichtung 126 dargestellt. In dieser Ausführung wird ein bandförmiges Testelement 112 eingesetzt, welches in Figur 2A im Detail dargestellt ist. Das bandförmige Testelement 112 weist ein Testband 210, beispielsweise ein Testband 210 aus Papier oder ähnlich saugfähigem Material, auf. Auf dem Testband 210 sind in beispielsweise regelmäßigen Abständen rechteckige Testbereiche 212 angeordnet, welche jeweils einen rechteckigen Rahmen 214 als Positionsreferenz, Längenskalen 216 als Skalierungsreferenzen, Referenzfelder 118 und ein zentral angeordnetes optisches Testfeld 114 aufweisen. Die Funktionsweise des bandförmigen Testelements 112 entspricht im Wesentlichen der Funktionsweise des in Figur 2 dargestellten Testelements 112.

Die Haltevorrichtung gemäß Figur 1A weist einen Vorratsbehälter 156 mit einem Ausgabeschlitz 158 und einen Abfallbehälter 160 mit einem Einzugsschlitz 162 auf Vorratsbehälter 156 und Abfallbehälter 160 sind über eine Brücke 164 miteinander verbunden, dergestalt, dass das bandförmige Testelement 112 vom Ausgabeschlitz 158 über die Brücke 164 zum Einzugsschlitz 162 befördert werden kann. Somit ist zwischen Ausgabeschlitz 158 und Einzugsschlitz 162 ein Sichtfenster 166 als Auswertungsposition ausgebildet, innerhalb dessen eine Probe auf das Testelement 112 appliziert und innerhalb dessen der Testbereich 212 zur Auswertung vom Sichtbereich 180 (in Figur 1 nicht dargestellt) einer Kamera 122 erfasst werden kann. Die Beförderung des bandförmigen Testelements 112 vom Vorratsbehälter 156 zum Abfallbehälter 160 kann beispielsweise mittels einer Handkurbel 168 erfolgen, mittels derer das bandförmige Testelement 112 weitergespult werden kann. Die Handkurbel kann am Vorratsbehälter 156 oder auch am Abfallbehälter 160 angeordnet sein. Anstelle einer Handkurbel 168 lassen sich auch andere Spulen- oder Beförderungsmechanismen einsetzen, beispielsweise eine motorbetriebene Spule oder ähnliches. Wie beispielsweise aus den Figuren 1B und 1C, welche die Haltevorrichtung 126 in Seitenansicht zeigen, ersichtlich, kann das Testelement 112 beispielsweise in dem Vorratsbehälter 156 und in dem Abfallbehälter 160 aufgewickelt gelagert werden beispielsweise durch ein Federelement vorgespannt (in den Figuren 1B und 1C nicht dargestellt).

In den Figuren 1B und 1C sind jeweils in Seitenansicht ein zweites und ein drittes Ausführungsbeispiel eines Systems 110 zum Auswerten eines Testelements 112 dargestellt, wobei Haltevorrichtungen 156 ähnlich der in Figur 1A dargestellten Haltevorrichtung 126 zum Einsatz kommen. Aus diesen Ausführungsbeispielen gemäß Figur 1B und 1C werden neben der Tatsache, dass bandförmige Testelemente 112 eingesetzt werden können insbesondere zwei weitere Aspekte der Erfindung deutlich. Zum einen wird ersichtlich, dass neben dem in Figur 1 dargestellten Fixieren des Mobiltelefons 120 relativ zum Testelement 112 auch ein freihändiges Positionieren (vgl. Figur 1B) oder ein einfaches Auflegen (vgl. Figur 1C) möglich ist. Die Tatsache, dass insbesondere bei der freihändigen Anordnung gemäß Figur 1B der Abstand zwischen Kamera 122 und Testelement 112 nicht definiert bzw. bekannt ist, lässt sich in dem oben beschriebenen Skalierungsschritt softwaretechnisch dadurch lösen, dass beispielsweise anhand der Längenskalen 216 (deren Dimension genau bekannt ist) ein von dem Testelement 112 aufgenommenes Bild reskaliert werden kann. Die unbekannte bzw. undefinierte Position des Testbereichs 212 im Bildbereich der Kamera 122 lässt sich beispielsweise durch eine Bilderkennungssoftware durch automatisches Erkennen des Rahmens 214 ausgleichen.

Zweitens wird aus den Ausführungsbeispielen gemäß den Figuren 1B und 1C deutlich, dass das Applizieren der Probe und die Auswertung durch die Kamera 122 nicht notwendigerweise von derselben Seite auf das Testelement 112 erfolgen muss, Verschiedene Möglichkeiten sind denkbar. So kann beispielsweise eine flüssige Probe (z. B. ein Blutstropfen) von einer Vorderseite 170 der Haltevorrichtung 126 her auf das Testelement 112 aufgebracht werden (Beispiel gemäß Figur 1C) oder von einer Rückseite 172 der Haltevorrichtung 126 (vgl. Beispiel gem. Figur 1B). In letzterem Fall kann, wie beispielsweise aus Figur 1B ersichtlich, die Brücke 164 ein Applikationsfenster 174 aufweisen, durch welches das Testelement 112 von der Rückseite 172 her zugänglich ist (die Applikation ist in Figur 1B und 1C symbolisch durch den Applikationspfeil 176 dargestellt). Weiterhin kann auch die Probenauswertung durch die Kamera 122 des Mobiltelefons 120 von der Vorderseite 170 oder von der Rückseite 172 her erfolgen. Auch eine Kombination einer Probenapplikation von einer Seite 170, 172 mit einer Auswertung von der jeweils anderen Seite 172, 170 ist möglich, beispielsweise indem das Testelement 112 transparent gewählt wird oder indem das Testelement 112 ein saugfähiges Material aufweist, durch welches die flüssige Probe diffundieren kann.

In Figur 1C ist ein Beispiel dargestellt, bei welchem die Auswertung von der Rückseite 172 her erfolgt. In diesem Fall weist die Brücke 164 eine Öffnung 178 als Sichtfenster auf, durch welches der gerade benutzte Testbereich 212 des Testelements 112 von der Kamera 122 beobachtet werden kann, dergestalt, dass der Testbereicht 212 im Sichtbereich 180 der Kamera 122 liegt. In dem Ausführungsbeispiel gemäß Figur 1C werden weiterhin die Oberflächen des Vorratsbehälters 156 und des Abfallbehälters 160 als Auflagefläche 182 für das Mobiltelefon 120 genutzt. Alternativ oder zusätzlich können auch separate Auflageflächen 182 oder zusätzliche Abstandshalter vorgesehen sein. Dabei kann, wie oben beschrieben, entweder das Mobiltelefon 120 auf die Haltevorrichtung 126 aufgelegt werden oder umgekehrt. Auch ein einfaches, freihändiges Halten des Mobiltelefons 120 gegen die Auflagefläche 182 ist denkbar.

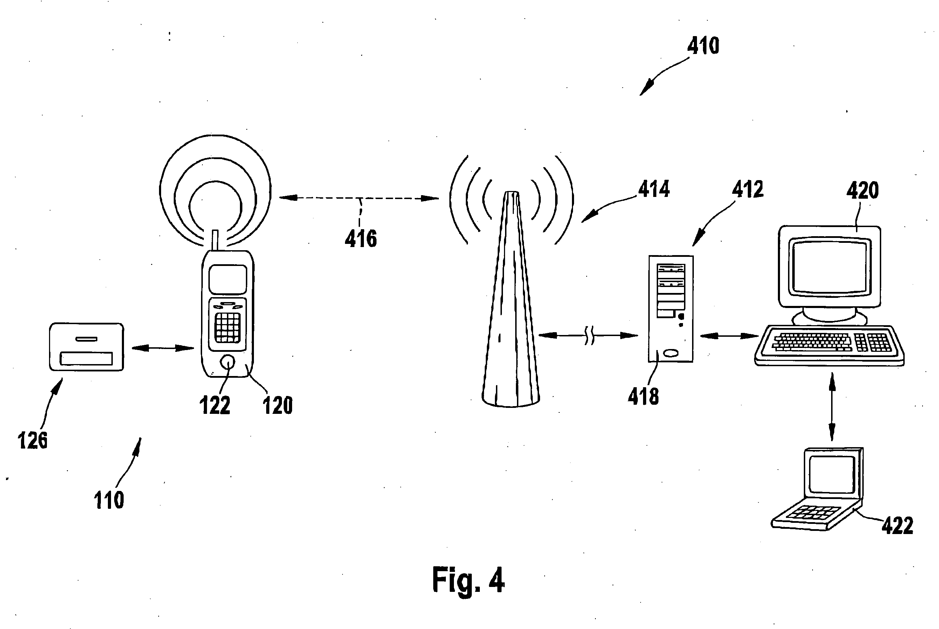

In Figur 4 ist ein viertes, bevorzugtes Ausführungsbeispiel eines erfindungsgemäßen Systems 410 zur Auswertung eines Testelements 112 dargestellt. In diesem Ausführungsbeispiel ist das System 110 gemäß dem in Figur 1 dargestellten Ausführungsbeispiel um einen Auswertungsserver 412 erweitert. Alternativ könnten auch die in den Figuren 1B oder 1C dargestellten Systeme 110 entsprechend erweitert werden. Der Auswertungsserver 412 ist mit einem Mobüfunknetzwerk 414 verbunden, welches in Figur 4 symbolisch dargestellt, ist. Auf diese Weise kann der Auswertungsserver 412 Daten mit dem Mobiltelefon 120 austauschen (gestrichelter Pfeil 416 in Figur 4).

Der Auswettungsserver 412 kann, wie in dem Ausführungsbeispiel gemäß Figur 4 dargestellt, mehrere miteinander verbundene Computersysteme 418, 420 aufweisen. Beispielsweise kann ein erstes Computersystem 418 für den Datenaustausch mit dem Mobilfunknetzwerk 414 (beispielsweise mittels eines Modems) benutzt werden und/oder zur Speicherung von Bildinfonnationen, die über das Mobilfunknetzwerk 414 übertragen werden. Weiterhin, kann das zweite Computersystem 420 zur Auswertung der Bildinformationen genutzt werden. Auch andere Ausgestaltungen sind selbstverständlich denkbar.

Weiterhin ist der Auswertungsserver 412 in diesem Ausführungsbeispiel gemäß Figur 4 mit einem Arztcomputer 422 verbunden (hier als Laptop 422 dargestellt). Ein Teil der Auswertung der Bildinformationen kann beispielsweise somit auch auf dem Arztcomputer 422 erfolgen und der Arztcomputer 422 kann Zugriff auf die (beispielsweise im ersten Computersystem 418 gespeicherten) Bildinformationen haben. Insbesondere kann auf dem Auswertungsserver 412 auch ein entsprechendes Datenbanksystem installiert sein, auf welches mit dem Arztcomputer 422 zugegriffen werden kann. Der Arztcomputer 422 muss nicht in räumlicher Nähe zu dem Auswertungsserver 412 angeordnet sein, sondern kann beispielsweise in einer Arztpraxis oder einem Home Office eines behandelnden Arztes angeordnet und beispielsweise über ein Modem oder eine VPN-Verbindung (Virtual Private Network) mit dem Auswertungsserver 412 verbunden sein.

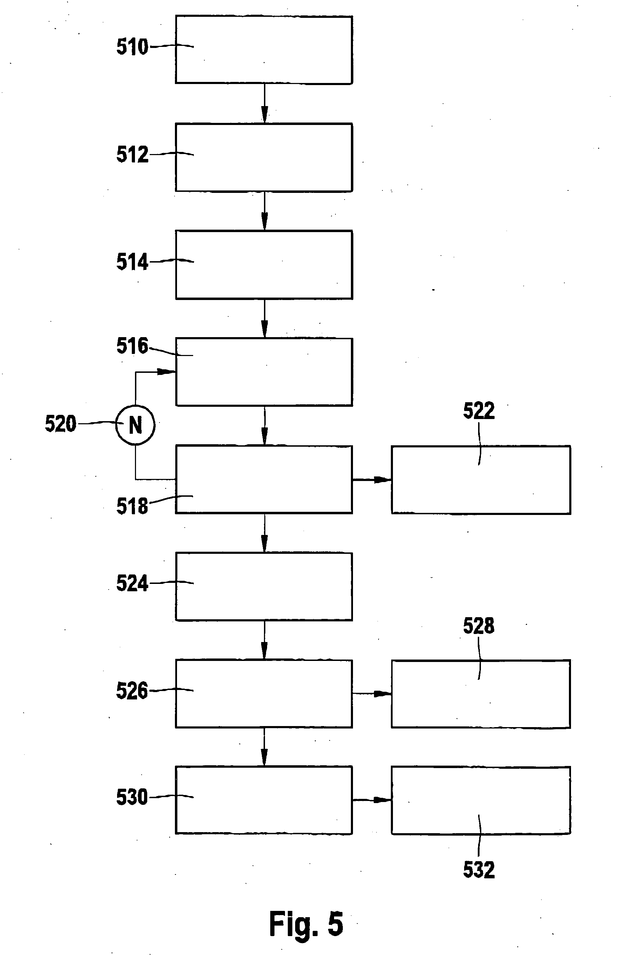

In Figur 5 ist ein Ausführungsbeispiel eines bevorzugten erfindungsgemäßen Verfahrens zur Auswertung eines Testelements 112 dargestellt, wobei im Weiteren angenommen werden soll, dass ein System 410 gemäß dem in Figur 4 dargestellten Ausführungsbeispiel eingesetzt wird. In Verfahrensschritt 510 wird eine Probe auf das Testelement 112 appliziert, beispielsweise ein Blutstropfen. Im Verfahrensschritt 512 wird das Testelement 112. in die Haltevorrichtung 126, insbesondere in eine Teststreifenaufnahme 138, eingebracht. Wie oben beschrieben, lassen sich auch andere Arten von Haltevorrichtungen 126 einsetzen. In Verfahrensschritt 514 wird die Haltevorrichtung 126, wie in Figur 1 dargestellt, auf ein Mobiltelefon 120 aufgesteckt. Alternativ könnte das Verfahren jedoch ein freihändiges Positionieren der Kamera 122 relativ zum Testelement 112 beinhalten (vgl. Figur 1B).

Anschließend wird in Verfahrensschritt 516 mittels der Kamera 122, gesteuert durch den Mikroprozessor 124, eine Bildinformation aufgenommen. Diese Bildinformation wird in Verfakewschritt 518 in einem Speicher des Mikroprozessors 124 gespeichert und gegebenenfalls vorverarbeitet, beispielsweise indem bereits eine vollständige oder teilweise Auswertung der Bildinformation erfolgt oder in dem eine Datenmenge der Bildinformation reduziert wird, beispielsweise durch Anwenden von Filteralgorithmen oder anderen Datenreduzierenden Algorithmen. Auch eine Reskalierung kann in diesem Schritt erfolgen. Die Verfahrewschritte 516 und 518 können im Bedarfsfall entsprechend wiederholt werden, beispielsweise mit einer Anzahl von N Wiederholungen, was in Figur 5 durch Bezugsziffer 520 gekennzeichnet ist. Auf diese Weise können beispielsweise zeitliche Entwicklungen der Verfärbung optischer Testfelder 114 beobachtet werden, beispielsweise in dem in regelmäßigen Zeitabständen (gesteuert durch den Mikroprozessor124) Bildinformationen aufgenommen werden.
Nach Aufnehmen der Bildinformation in Verfahrensschritt 516 und gegebenenfalls einer Auswertung kann optional in Verfahrensschritt 522 eine Ausgabe der Analysenergebnisse erfolgen, beispielsweise eine Ausgabe auf dem Display 125 des Mobiltelefons 120 in Form einer Darstellung der gemessenen Blutglukosekonzentration. Alternativ oder zusätzlich kann in Verfahrensschritt 524 die in Verfahrensschritt 516 aufgenommene Bildinformation oder gegebenenfalls aus dieser Bildinformation abgeleitete Informationen und/oder Zusatzinformationen über das Mobilfunknetz 414 an den Auswertungsserver 412 übertragen werden. Diese Übertragung kann verschiedenartig ausgestaltet sein und kann auch zusätzliche Schritte enthalten, beispielsweise einen Authentifizierungsschritt, bei welchem vom Mobiltelefon 120 Authentifizierungsdaten an den Auswertungsserver 412 übertragen werden, anhand dessen der Auswertungsserver 412 erkennen kann, welcher Systemnutzer die Daten übersendet. Auch entsprechende Passwörter können mitgeschickt werden, um zu verhindern, dass ein unberechtigter Zugriff erfolgt.

Anschließend erfolgt in Verfahrensschritt 526 auf dem Auswertungsserver 412 eine weitere Auswertung der übertragenen Bildinformation oder der weiteren übertragenen Daten, wobei beispielsweise wiederum ein Analysenergebnis berechnet wird, die Daten in eine Datenbank eingetragen werden oder ähnliches. Die dabei gewonnenen Daten und/oder die Bildinformationen können in Verfahrensschritt 528 an den Arztcomputer 422 übermittelt werden oder es kann eine Mitteilung an den Arztcomputer 422 übermittelt werden, dass neue Patientendaten vorliegen. Anschließend können in Verfahrensschritt 530 Ergebnisse des Auswertungsschritts 526 über das Mobilfunknetz 414 zurück an das Mobiltelefon 120 übertragen werden, beispielsweise in Form einer einfachen SMS (Short Message Service), wo diese wiederum beispielsweise in einem Datenspeicher des Mikrocomputers 124 gespeichert werden können und/oder in Verfahrensschritt 532 auf dem Display 125 für den Benutzer lesbar ausgegeben werden können.

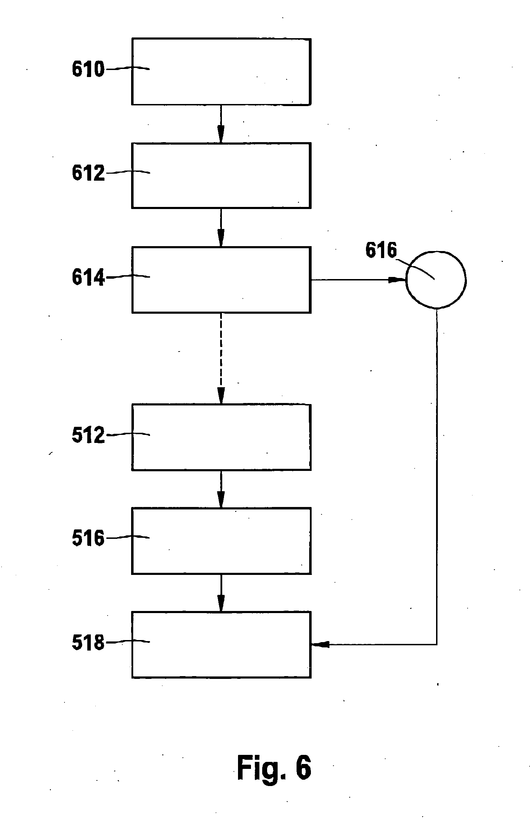

In Figur 6 ist eine Variante des in Figur 5 dargestellten Verfahrens schematisch und auszugsweise dargestellt, wobei das erfindungsgemäße Verfahren durch einen Kalibriervorgang ergänzt ist. Zu diesem Zweck wird zunächst in Verfahrensschritt 610 ein Kalibrierelement 310 in die Teststreifenaufnahme 138 der Haltevorrichtung 126 eingeschoben. Anschließend wird in Verfahrensschritt 612, gesteuert durch den Mikroprozessor 124, mittels der Kamera 122 eine Bildinformation des Kalibrierelements 310 aufgenommen. Aus dieser Bildinfonnation wird in Verfahrensschritt 614 ein Kalibrierfaktor bestimmt, welcher beispielsweise die Eigenschaften der Lichtquelle 150, Chargeninformationen des Teststreifens 112, Abbildungseigenschaften der Kamera 122 oder ähnliches berücksichtigt. Diese Berechnung des Kalibrierfaktors erfolgt beispielsweise dadurch, dass die Bildinformation, die in Schritt 612 gewonnen wurde, mit einer bekannten Verfärbung, bekannte Reflexionseigenschaften o. ä. der optischen Kalibrierfelder 312 des Kalibrierelements 310 verglichen wird. Bei dem Kalibrierfaktor muss es sich nicht notwendigerweise um einen einfachen Faktor handeln, sondern es kann sich beispielsweise auch um ein Vektor oder eine Matrix handeln, so dass beispielsweise auch laterale Inhomogenitäten der Abbildungseigenschaften der Kamera 122 berücksichtigt werden können. Der Kalibrierfaktor wird in Verfahrensschritt 616 abgespeichert, beispielsweise in einem Datenspeicher des Mikroprozessors 124. Damit ist die Kalibrierroutine, welche in diesem Ausführungsbeispiel die Schritte 610, 612, 614 und 616 umfasst, im Wesentlichen abgeschlossen. Die gesamte Kalibrierroutine kann beispielsweise durch ein entsprechendes Softwaremodul, welches auf dem Mikrocomputer 124 des Mobiltelefons 120 ausgeführt wird, gesteuert werden.

Anschließend werden die Verfahrensschritte 512, 516 und 518, welche oben bereits beschrieben wurden, durchgeführt, wobei wiederum ein Teststreifen 112 mit einer applizierten Probe in die Haltevorrichtung 126 eingebracht wird (Schritt 512), anschließend eine Bildinformation aufgenommen wird (Schritt 516) und schließlich diese Bildinformation ganz oder teilweise ausgewertet wird (Schritt 518), Bei der Auswertung in Schritt 518 wird der in Verfahrensschritt 616 abgespeicherte Kalibrierfaktor berücksichtig, beispielsweise in dem Messergebnisse mit dem Kalibrierfaktor multipliziert werden. Die weiteren Verfahrensschritte können beispielsweise gemäß dem Ausführungsbeispiel in Figur 5 durchgeführt werden. Alternativ oder zusätzlich kann bei der Übertragung der Bildinformation in Verfahrensschritt 524 an den Auswertungsserver 412 auch der Kalibrierfaktor mit übermittelt werden, so dass dieser Kalibrierfaktor beispielsweise auch bei der Auswertung in Verfahrensschritt 526 auf dem Auswertungsserver 412 berücksichtigt werden kann.

### Bezugszeichenliste

- 110: System zur Auswertung eines Testelements
- 112: Testelements
- 114: optische Testfelder
- 116: Positionsmarken
- 118: Referenzfelder
- 120: Mobiltelefon
- 122: Kamera
- 123: Tastatur
- 124: Mikroprozessor
- 125: Display
- 126: Haltevorrichtung
- 128: Aufklemmschiene
- 130: Schnittstelle
- 132: Schnittstelle
- 134: Einschubschlitz
- 136: Gehäuse
- 138: Teststreifensaufnahme
- 140: Andruckfelder
- 142: Andruckstempel
- 144: Halterung
- 146: Anschlag
- 148: Aussparung
- 150: Lichtquelle
- 152: Streulichtblende
- 154: Linse
- 156: Vorratsbehälter
- 158: Ausgabeschlitz
- 160: Abfallbehälter
- 162: Einzugsschlitz
- 164: Brücke
- 166: Sichtfenster
- 168: Handkurbel
- 170: Vorderseite
- 172: Rückseite
- 174: Applikationsfenster
- 176: Probenapplikation
- 178: Sichtfenster
- 180: Sichtbereich
- 182: Auflagefläche

- 210: Testband
- 212: Testbereich
- 214: Rahmen
- 216: Längenskala

- 310: Kalibrierelement
- 312: optische Kalibrierfelder

- 410: System zur Auswertung eines Testelements
- 412: Auswertungsserver
- 414: Mobiliunknetzwerk
- 416: drahtloser Datenaustausch
- 418: Computersystem
- 420: Computersystem
- 422: Arztcomputer

- 510: Applizieren der Probe auf Testelement
- 512: Einbringen des Testelements in Haltevorrichtung
- 514: Aufstecken der Haltevorrichtung auf Mobiltelefon
- 516: Aufnehmen der Bildinformation
- 518: Speicherung und Vorverarbeiten
- 520: Wiederholung
- 522: Ausgabe auf Display
- 524: Übertragung an Auswertungsserver
- 526: Auswertung
- 528: Übermittlung an Arztcomputer
- 530: Übermittlung an Mobiltelefon
- 532: Ausgabe
- 610: Einbringen eines Kalibfierelements in die Haltevorrichtung
- 612: Aufnahme einer Bildinformation
- 614: Berechnung Kalibrierfaktor
- 616: Abspeichern des Kalibrierfaktors

## Patentansprüche

1. Haltevorrichtung (126) zur Auswertung eines Testelements (112) mit mindestens einer Halterung (138) zur Aufnahme mindestens eines mindestens ein optisches Testfeld (114) aufweisenden Testelements (112), wobei die Haltevorrichtung (126) derart ausgestaltet ist, dass das Testelement (112) in mindestens einer Auswertungsposition (166; 178) positionierbar ist, wobei in der mindestens einen Auswertungsposition (166; 178) das mindestens eine optische Testfeld (114) von einem Sichtbereich (180) mindestens einer Kamera (122) eines mobilen Datenübertragungsgerätes (120) erfassbar ist

2. Haltevorrichtung (126) gemäß dem vorhergehenden Anspruch, **gekennzeichnet durch** mindestens eine Verbindungseinrichtung (128; 182) zur räumlichen Fixierung und/oder Positionierung der Haltevorrichtung (126) zu der mindestens einen Kamera (122) des mobilen Datenübertragungsgerätes (120), wobei die räumliche Fixierung derart ausgestaltet ist, dass in mindestens einer Position des mindestens einen Testelements (112) der Sichtbereich (180) der mindestens einen Kamera (122) mindestens ein optisches Testfeld (114) des mindestens einen Testelements (112) erfasst.

3. Haltevorrichtung (126) gemäß einem der beiden vorhergehenden Ansprüche mit zusätzlich mindestens einem Magazin (156, 160) zur Aufnahme und/oder Ausgabe mindestens eines Testelements (112).

4. Haltevorrichtung (126) gemäß einem der beiden vorhergehenden Ansprüche mit zusätzlich mindestens einer Lichtquelle (150) zur Beleuchtung mindestens eines Teilbereichs mindestens eines Testelements (112), insbesondere einer Lichtquelle, (150) zur Emission von sichtbarem und/oder infrarotem und/oder ultraviolettem Licht.

5. System (110; 410) zur Auswertung eines Testelements (112) mit:
a) mindestens einem mindestens ein optisches Testfeld (114) aufweisenden Testelement (112); und
b) mindestens einem, mindestens eine Kamera (122) mit einem Sichtbereich (180) aufweisenden mobilen Datenübertragungsgerät (120).

6. System (110; 410) gemäß dem vorhergehenden Anspruch, mit zusätzlich;
c) mindestens einer Haltevorrichtung (126) gemäß einer der vorhergehenden, auf eine Haltevorrichtung (126) gerichteten Ansprüche.

7. System (110; 410) gemäß einem der beiden vorhergehenden Ansprüche, **dadurch gekennzeichnet, dass** durch Aufbringen mindestens einer vorgegebenen chemischen Substanz auf das mindestens eine Testelement (112) mindestens eine optische Eigenschaft, insbesondere eine Farbe und/oder eine Reflektivität und/oder eine Fluoreszenzeigewchaft, des mindestens einen optischen Testfelds (114) des mindestens einen Testelements (112) verändert wird.

8. System (110; 410) gemäß einem der vorhergehenden, auf ein System (110; 410) gerichteten, Ansprüche, **dadurch gekennzeichnet, dass** das mindestens eine Testelement (112) mindestens zwei optische Testfelder (114) aufweist.

9. System (110; 410) gemäß einem der vorhergehenden, auf ein System (110; 410) gerichteten Ansprüche, **dadurch gekennzeichnet, dass** das mindestens eine Testelement (112) mindestens eine Referenzeinrichtung (116, 118; 214, 216) zur Erzeugung mindestens einer Referenz, insbesondere einer Farbreferenz und/oder einer Fluoreszenzreferenz und/oder einer Reflexionsreferenz und/oder einer Positionsreferenz und/oder einer Skalierungsreferenz, aufweist.

10. System (110; 410) gemäß einem der vorhergehenden, auf ein System (110; 410) gerichteten Ansprüche, mit zusätzlich:
d) mindestens einem Auswertungsserver (412), wobei der mindestens eine Auswertungsserver (412) folgendes aufweist:
- Mittel (418) zum Empfangen mindestens einer Information, vorzugsweise der mindestens einen Bildinformation, von dem mindestens einen mobilen Datenübertragungsgerät (120);
- Mittel (420) zum Analysieren der mindestens einen Information, insbesondere Mittel (420) zum Berechnen einer Konzentration mindestens einer vorgegebenen chemischen Substanz; und
- Mittel (418) zum Übersenden mindestens eines Analyseergebnisses an das mindestens eine mobile Datenübertragungsgerät (120).

11. Testelement (112) mit mindestens einem optischen Testfeld (114) und mindestens einer Referenzeinrichtung (116, 118; 214, 216) zur Erzeugung mindestens einer Referenz, vorzugsweise mindestens einer der folgenden Referenzen: eine Farbreferenz; eine Fluoreszenzreferenz; eine Reflexionsreferenz; eine Positionsreferenz; eine Skalierungsreferenz.

12. Verfahren zur Auswertung eines Testelements (112) mit folgenden Verfahrensschritten:
a) mindestens eine Probe, vorzugsweise eine flüssige Probe, wird mit mindestens einem mindestens ein optisches Testfeld (114) aufweisenden Testelement (112) in Kontakt gebracht; und
b) mittels einer Kamera (122) eines mindestens eine Kamera (122) mit einem Sichtbereich (180) aufweisenden mobilen Datenübertragungsgerätes (120) wird mindestens eine Bildinformation des Testelements (112) aufgenommen.

13. Verfahren gemäß dem vorhergehenden Anspruch mit zusätzlich folgendem Verfahrensschritt:
c) das mobile Datenübertragungsgerät (120) wird mit mindestens einer Haltevorrichtung (126) gemäß einem der vorhergehenden, auf eine Haltevorrichtung gerichteten Ansprüche in Verbindung gebracht, dergestalt, dass der Sichtbereich (180) mindestens ein optisches Testfeld (114) des mindestens einen Testelements (112) erfasst.

14. Verfahren gemäß einem der beiden vorhergehenden Ansprüche mit zusätzlich folgendem Schritt:
d) in einem Skalierungsschritt wird die mindestens eine Bildinformation mittels mindestens einer bekannten, aus der mindestens einen Bildinformation generierten Skalierung des mindestens einen optischen Testfeldes (114) reskaliert.

15. Verfahren gemäß einem der vorhergehenden Ansprüche, **dadurch gekennzeichnet, dass** mindestens ein Auswertungsergebnis ausgegeben wird.

16. Verfahren gemäß einem der vorhergehenden Verfahrensansprüche mit zusätzlich folgendem Verfahrensschritt:
e) die mindestens eine Information, wobei die mindestens eine Information vorzugsweise die mindestens eine Bildinformation umfasst, wird von dem mobilen Datenubertragungsgerät (120) an mindestens einen Auswertungsserver (412) übennittelt.

17. Verfahren gemäß einem der beiden vorhergehenden Verfahrensansprüche mit zusätzlich folgenden Schritten:
f) die mindestens eine Information wird mittels eines zweiten Auswertungsverfahrens von dem mindestens einen Auswertungsserver (412) analysiert; und
g) ein Analyseergebnis wird von dem mindestens einen Auswertungsserver (412) an das mobile Datenübertragungsgerät (120) übermittelt

18. Verfahren gemäß einem der vorhergehenden Verfahrensansprüche mit zusätzlich folgenden Schritten:
h) mittels der mindestens einen Kamera (122) wird mindestens eine Kalibrier-Bildinformation mindestens eines Kalibrierelements (310) gemäß einem der vorhergehenden, auf ein Kalibrierelement (310) gerichteten Ansprüche aufgenommen.
